# EUROPÄISCHE PATENTANMELDUNG

(11) **EP 4 458 990 A1**
(43) Veröffentlichungstag der Anmeldung: **06.11.2024**
(21) Anmeldenummer: 24173359.1
(22) Anmeldetag: 30.04.2024
(51) Int. Cl.: C12Q 1/689, C12Q 1/06

(54) **VERFAHREN ZUR BESTIMMUNG DER LEBENDZELLZAHLEN VON PROZESSRELEVANTEN (PROZESSSTÖRENDEN) MIKROORGANISMEN IN EINER PROBE**

(30) Priorität: 02.05.2023 DE 102023111232
(71) Anmelder: DyeNA Genetics GmbH, 97422 Schweinfurt (DE)
(72) Erfinder: BENKERT, Thomas, 97422 Schweinfurt (DE)
(74) Vertreter: Gleim, Christian Ragnar

(57) **Zusammenfassung**

Verfahren zur Bestimmung der Lebendzellzahlen von prozessrelevanten Mikroorganismen in einer Probe, wobei die Probe mittels quantitativer Real-Time PCR untersucht wird.

## Beschreibung

Die Erfindung betrifft ein Verfahren zur Bestimmung der Lebendzellzahlen von prozessrelevanten (prozessstörenden) Mikroorganismen mittels quantitativer Real Time PCR Technologie. Auch prozessstörende Mikroorganismen sind in diesem Zusammenhang als prozessrelevant anzusehen. Die zu untersuchenden Proben können beispielsweise aus Oberflächengewässern, aus Brauchwässern oder aus Abwässern stammen. Insbesondere kann es sich um Proben einer Industrieanlage oder einer Kläranlage handeln, beispielsweise aus dem Zulauf, dem Ablauf, dem Belebungsbecken, den SBR-Reaktoren (SBR: Sequencing Batch Reactor) oder den Faultürmen. Auch eine Untersuchung von Proben aus Biogasanlagen ist in diesem Zusammenhang denkbar. Bei den jeweils relevanten Prozessen kann es sich insbesondere um die mikrobiologische Eliminierung, beispielsweise um den biologischen Stickstoffabbau, die mikrobiologische Umsetzung (Metabolisierung) organischer und anorganischer Stoffe, die Optimierung und Überwachung von Betriebsprozessen sowie um die Vermeidung von Prozessstörungen handeln. Dazu können Untersuchungen des Schwimm- und Blähschlamms sowie hinsichtlich der Verwertung der organischen Biomasse notwendig sein.

Zur Beurteilung der Abbauleistung und der Funktionalität der mikrobiologischen Prozesse von beispielsweise Kläranlagen oder Biogasanlagen ist es wichtig, die Lebendzellzahl der entsprechenden prozessrelevanten (prozessstörenden) und prozessstörenden Mikroorganismen zu bestimmen. So ist beispielsweise eine ausreichende Lebendzellzahl an Stickstoff-abbauenden Bakterien (Nitrifikanten und Denitrifikanten) notwendig, um Ammonium möglichst vollständig entfernen zu können, ohne dass Stickstoff-haltige Abbauprodukte (beispielsweise Nitrite oder Nitrate) zurückbleiben.

Der Nachweis von Bakterien kann insbesondere über Mikroskopie und/oder mit FISH-Technologie erfolgen. Im Vergleich zu einer quantitativen Real-Time PCR ist die Präzision und Spezifität der FISH-Technologie dabei nicht allzu hoch. Darüber hinaus sind die Untersuchungsmethoden verhältnismäßig zeitaufwändig.

Als Polymerase-Kettenreaktion (polymerase chain reaction, kurz PCR) bezeichnet man Methoden zur Vervielfältigung von spezifischen Genabschnitten mittels des. Enzyms DNA-Polymerase. Da es sich um eine Kettenreaktion handelt, können die Produkte vorheriger Zyklen als Ausgangsstoffe für den nächsten Zyklus dienen, so dass eine exponentielle Vervielfältigung möglich ist. Bei einer quantitativen Real Time PCR (qPCR) wird zusätzlich zu den beiden Primern eine weitere, zielgenspezifische fluoreszenzmarkierte Gensonde verwendet. Bei dem Vervielfältigungsprozess wird die Gensonde abgebaut und das gebundene Fluorophor freigesetzt. Durch das entstehende Fluoreszenzsignal, das mit jedem Zyklus ansteigt, wird die ursprüngliche Zielgenmenge in der Ausgangsprobe quantifiziert.

In medizinischen und biologischen Laboratorien wird die qPCR beispielsweise zur Erkennung von Erbkrankheiten und Virusinfektionen eingesetzt. Die qPCR ist schnell, hochspezifisch und extrem sensitiv. Darüber hinaus sind die Ergebnisse einfach zu interpretieren.

Eine qPCR wird eingesetzt, um einen kurzen, genau definierten Abschnitt eines DNA-Stranges zu vervielfältigen. Dabei kann es sich um ein Gen, ein Teil eines Gens oder auch um nichtcodierende DNA-Sequenzen handeln.

Eine qPCR benötigt grundsätzlich mehrere Komponenten:
- die Original-DNA, die den zu vervielfältigenden Ziel-Abschnitt enthält
- zwei Primer, um auf den beiden Einzelsträngen der DNA jeweils den Startpunkt der DNA-Synthese festzulegen, wodurch der zu vervielfältigende Bereich von beiden Seiten begrenzt wird
- eine fluoreszenzmarkierte Gensonde (Fluorophor und Quencher) für die Generierung eines Fluoreszenzsignal das für die Quantifizierung benötigt wird
- DNA-Polymerase, um den festgelegten Abschnitt zu kopieren
- Desoxyribonukleosidtriphosphate als Bausteine für den von der DNA-Polymerase synthetisierten DNA-Strang
- Magnesiumionen, die für die Funktion der Polymerase essentiell sind, die Anlagerung der Primer stabilisieren und lösliche Komplexe mit den Desoxyribonucleosidtriphosphaten bilden
- Pufferlösungen, die eine für die DNA-Polymerase geeignete chemische Umgebung sicherstellen.

Entsprechende Systeme werden als fertige Testsysteme von zahlreichen Unternehmen angeboten, so dass nur noch die zu untersuchende DNA zugegeben werden muss.

Der qPCR-Prozess besteht aus mehreren Zyklen. Jeder Zyklus besteht dabei aus den folgenden drei Schritten:
1. Denaturierung: Die doppelsträngige DNA wird erhitzt, um die Stränge zu trennen.
2. Primerhybridisierung: Die Temperatur wird so abgesenkt, dass eine Anlagerung der Primer an den Einzelstrang der DNA möglich ist.
3. Elongation: Die fehlenden Stränge werden von der DNA-Polymerase mit freien Nukleotiden aufgefüllt. Bei diesem Schritt wird die fluoreszenzmarkierte Gensonde bei einer qPCR durch das Taq-Enzym abgebaut. Hierdurch entsteht das Fluoreszenzsignal für die Quantifizierung.

qPCR-Analysen können beispielsweise zum Nachweis von Krebszellen oder von anderen Mutationen eingesetzt werden. Darüber hinaus werden diese zunehmend auch im Bereich der Lebensmittelanalytik eingesetzt, beispielsweise zur Prozesskontrolle oder zur Identifizierung von Pathogenen. Auch ein Einsatz im Bereich der Futtermittelanalytik ist bereits bekannt.

Darüber hinaus ist es möglich, lediglich die DNA lebender Zellen nachzuweisen. Dazu erfolgt eine Behandlung der Probe mit speziellen photoreaktiven Reagenzien, durch die die DNA toter Zeller blockiert wird. Diese Art der qPCR wird auch Viability PCR (v-PCR, Lebensfähigkeits-PCR) genannt.

Ausgehend von diesem vorbekannten Stand der Technik lag der Erfindung die Aufgabe zugrunde, ein verbessertes Verfahren zur Bestimmung der Lebenzellzahlen von prozessrelevanten (prozessstörenden) Mikroorganismen in einer Probe anzugeben, das möglichst rasch und zuverlässig Ergebnisse liefert.

Erfindungsgemäß wird die Probe mittels einer quantitativen Real-Time PCR (qPCR) untersucht. Dadurch ist eine rasche und kostengünstige Analyse der entsprechenden Proben möglich. Der Einsatz von quantitativen Real-Time PCR-Analysen ist zwar bereits aus verschiedenen Bereichen bekannt, allerdings wurde in diesem Zusammenhang bislang keine Untersuchungen von prozessrelevanten (prozessstörenden) Mikroorganismen einer Probe aus einer beispielsweise Kläranlage oder Biogasanlage durchgeführt. Der Anstieg der Fluoreszenz kann dabei im Labor in Echtzeit am PCR-Cycler beobachtet werden.

Bei einer quantitativen Real-Time PCR (Real-Time qPCR) wird eine fluoreszenzmarkierte Gensonde neben den beiden Primern eingesetzt.

Die quantitative Real-Time PCR kann beispielsweise auch als digitale PCR (dPCR) oder als Reverse Transkriptase qPCR (RT qPCR) durchgeführt werden. In diesem Fall wird die Lebendzellzahl anhand von Quantifizierungsstandards oder statistischer Methoden bestimmt. Die Zahl der Zyklen ist wichtig für die qPCR, sie liegt immer zwischen 40 - 45 Zyklen. Ist die Zyklenzahl zu kurz, entsteht kein Signal. Außerdem wird anhand der Zyklenzahl die Probe quantifiziert. Der CT Wert genannt ist jener Zyklus (Cycle) bei dem das Fluoreszenzsignal den Background (Threshold) übersteigt. Der CT Wert wird auch alternativ Cq für Cycle quantification genannt. Darüber hinaus wird in der Regel Taq-Polymerase verwendet, da diese bei hohen Temperaturen eine erhöhte Stabilität aufweist.

Auf diese Weise kann eine Gewässeranalytik beispielsweise in Oberflächenwasser, in Abwasser, in Kanalsystemen oder auch in verschiedenen Bereichen von Kläranlagen oder Industrieanlagen möglich werden, bei der der Gehalt an prozessrelevanten (prozessstörenden) Mikroorganismen rasch ermittelt werden kann.

Um ermitteln zu können, ob die prozessrelevanten (prozessstörenden) Mikroorganismen tatsächlich leben und somit aktiv sind, kann vorzugsweise eine Viability-qPCR (Lebensfähigkeits-qPCR) durchgeführt werden. Auf diese Weise kann sichergestellt werden, dass der ermittelte Gehalt an Mikroorganismen tatsächlich dem Gehalt an lebenden Mikroorganismen entspricht. Da lediglich die lebenden und damit die aktiven Mikroorganismen zum Abbau von Schadstoffen beitragen, sind lediglich die lebenden Mikroorganismen relevant. Die toten Mikroorganismen bauen dagegen keine Schadstoffe mehr ab, so dass diese für die Beurteilung der Prozesse von geringerer Relevanz sind. Dazu kann die Probe vorzugweise mit einem Farbstoff, insbesondere mit PMA oder mit EMA versetzt werden.

Bei der Vermeidung von Prozessstörungen sind insbesondere fadenförmige Bakterien wie beispielsweise Microthrix parcivella , Thiothrix I und Thiothrix II oder Fadenorganismen Typ 021N zu beachten. Diese sind häufig die Ursache für Schaum- und Schwimmschlamm in Kläranlagen. Auch Methanogene können in den Faultürmen von Kläranlagen oder in Biogasanlagen zu Störungen zu Prozessstörungen führen, wenn diese in zu geringer Anzahl vorhanden sind.

Vorzugsweise kann mit dem erfindungsgemäßen Verfahren der Gehalt an Stickstoff-abbauenden Bakterien untersucht werden. Eine ausreichende Lebendzellzahl an Stickstoff-abbauenden Bakterien ist notwendig, um Stickstoff-haltige Abbauprodukte wie beispielsweise Ammonium, Nitrite oder Nitrate möglichst vollständig entfernen zu können. Von Bedeutung sind in diesem Zusammenhang insbesondere Ammoniumoxidierende Bakterien (AOB) und Nitrit-oxidierende Bakterien (NOB), beispielsweise Candidatus Nitrotoga oder Anammox-Bakterien.

Insbesondere kann mit dem erfindungsgemäßen Verfahren der Gehalt an Nitrifikanten (beispielsweise AOB, NOB und Nitrotoga) untersucht werden. Bei Nitrifikanten handelt es sich um Bakterien, mittels derer eine Nitrifikation erreicht wird. Nitrifikation ist die biologische Oxidation von Ammoniak zu Nitrit (insbesondere durch AOB), gefolgt von der Oxidation des Nitrits zu Nitrat (insbesondere durch NOB und Nitrotoga). Die Umwandlung von Ammonium zu Nitrit ist dabei in der Regel der geschwindigkeitsbestimmende und damit der essentielle Schritt. Ist der Gehalt von Nitrifikanten zu gering, ist die Nitrifikation nicht immer vollständig, so dass gegebenenfalls ein zu hoher Gehalt an Ammonium, Nitrit und/oder Nitrat im Abwasser verbleibt.

Alternativ oder zusätzlich dazu kann mit dem erfindungsgemäßen Verfahren der Gehalt an Denitrifikaten bestimmt werden. Bei Denitrifikanten handelt es sich um Bakterien, mittels derer eine Denitrifikation erreicht wird. Denitrifikation ist ein mikrobiell unterstützter Prozess, bei dem Nitrat reduziert und als Endprodukte molekularer Stickstoff und Sauerstoff entstehen. Dabei wird Nitrat zunächst zu Nitrit reduziert, anschließend zu Stickoxid und Distickstoffoxid, bevor molekularer Stickstoff gebildet wird. Dieser Prozess findet unter anaeroben Bedingungen statt. Da durch die Denitrifikation Nitrat entfernt werden kann, wird diese in Kläranlagen zur Behandlung von Abwässern mit hohem Stickstoffgehalt eingesetzt. Allerdings entsteht bei der Denitrifikation Distickstoffoxid als Zwischenprodukt, bei dem es sich um ein Treibhausgas handelt. Daher soll sichergestellt werden, dass ausreichend denitrifizierende Bakterien vorhanden sind, um die Denitrifikation vollständig ablaufen zu lassen.

Ein zu hoher Gehalt an fadenförmigen Bakterien wie beispielsweise Microthrix, Thiothrix oder Chloroflexi kann zu einer Bildung von Bläh- und Schwimmschlamm führen, was Prozessstörungen nach sich zieht. Auch der Gehalt an Bio-P-Mikroorganismen ist relevant. Dabei handelt es sich um spezialisierte Polyphosphatakkumulierende Mikroorganismen, die dem Abwasser Phosphat entziehen. In Faultürmen und Biogasanlagen sind darüber hinaus Methanogene von Bedeutung. Durch die Bestimmung der Lebendzellzahl kann hier die Aktivität der Methanogenen und somit die Biogasproduktion messen. Insofern kann auch die Bestimmung dieser Lebendzellzahlen von Bedeutung und damit von Interesse sein.

Bei der wässrigen Probe kann es sich um eine wässrige Lösung oder um eine wässrige Suspension handeln. Abhängig von der Art der wässrigen Probe kann zunächst eine Abtrennung der enthaltenen Feststoffe erforderlich sein, grundsätzlich sind jedoch bei der Entnahme der Probe aus dem Gewässer keine besonderen Vorkehrungen erforderlich.

Um eine Abwasserprobe aus dem Belebungsbecken zu untersuchen, kann beispielsweise folgendes Verfahren eingesetzt werden:
- Die wässrige Probe wird auf einen Trockensubstanzwert (TS) von 0,5 bis 5.0 g/l, insbesondere auf 2,0 g/l, verdünnt. Insgesamt sollt ein Probenvolumen von 1000 µl erhalten werden.
- 500 µl der Probe werden für eine Prozesskontrolle bei 70 °C für insgesamt 10 Minuten inkubiert.
- 160 µl der Probe werden entnommen und bei 13.000 rpm pelletiert.
- Der Überstand wird abgenommen und in 500 µl Pufferlösung aufgenommen.
- 8 µl PMA Stocklösung wird zugegeben.
- Die Probe wird 10 min bei Dunkelheit inkubiert.
- Die Probe wird 15 min bei maximaler Leuchtstärke bestrahlt, dabei wird die Probe alle 3 Minuten kurz geschwenkt.
- Die Probe wird bei 13.000 rpm pelletiert.
- Der Überstand wird abgenommen und in 160 µl Wasser aufgenommen.
- 150 µl der so vorbereiteten Probe werden für die DNA Extraktion eingesetzt.

Die benötigte PMA Stocklösung sollte eine Konzentration von 0,5 mM bis 5,0mM haben. Für das oben genannte Verfahren wurde eine PMA-Stocklösung mit einer Konzentration von 2,5 mM eingesetzt.

PMA (propidium monoazide (3-Amino-8-azido-5-[3-(diethylmethylammonio)propyl]-6-phenylphenanthridium dichloride, Propidium monoazide dichloride)) und EMA (Ethidiumbromid-monoazid alternativ: 3-Amino-8-azido-5-ethyl-6-phenylphenanthridiniumbromid) sind hochaffine photoreaktive DNA-bindende Farbstoffe, die insbesondere bei der Viability-PCR (Lebensfähigkeits-PCR) eingesetzt werden. Die Farbstoffe sind schwach fluoreszierend, nach der Bindung an Nukleinsäuren wird der Farbstoff jedoch stärker fluoreszierend. Die Farbstoffe ist zellmembranundurchlässig und können daher selektiv verwendet werden, um nur die exponierte DNA aus toten Zellen zu modifizieren. EMA und PMA binden an die freie in der Lösung befindliche DNA der toten Bakterien. Durch die Zellmembran ist die DNA der lebenden Bakterien für PMA und EMA nicht zugänglich. PMA und EMA bindet an die freie in der Lösung befindliche DNA und wird durch die UV-Bestrahlung fest an die DNA gebunden. Hierdurch wird die DNA der toten Bakterien durch PMA und EMA sterisch blockiert. Die Taq-Polymerase ist nicht in der Lage, die sterisch blockierte DNA zu vervielfältigen und wird somit ausgeschlossen. Die Taq-Polymerase kann demnach nur die DNA von lebenden Bakterien vervielfältigen, da diese nicht blockiert ist. Somit werden nur Gene von lebenden Bakterien mit der qPCR detektiert und quantifiziert. Dadurch ist ein selektiver Nachweis lebensfähiger Zellen durch quantitative Echtzeit-PCR auch in Gegenwart von toten Zellen möglich.

## Patentansprüche

1. Verfahren zur Bestimmung der Lebendzellzahlen von prozessrelevanten Mikroorganismen in einer Probe,
- **dadurch gekennzeichnet, dass**
- die Probe mittels quantitativer Real-Time PCR untersucht wird.

2. Verfahren nach Anspruch 1,
- **dadurch gekennzeichnet, dass**
- der Gehalt an für Kläranlagen relevanten Bakterien untersucht wird.

3. Verfahren nach Anspruch 1 oder 2,
- **dadurch gekennzeichnet, dass**
- die Probe mittels einer Viability-PCR untersuchst wird.

4. Verfahren nach Anspruch 3,
- **dadurch gekennzeichnet, dass**
- die Probe mit einem Farbstoff, insbesondere mit PMA oder EMA, versetzt wird.

5. Verfahren nach Anspruch 4,
- **dadurch gekennzeichnet, dass**
- die Probe nach der Zugabe des Farbstoffs inkubiert wird.

6. Verfahren nach einem der vorstehenden Ansprüche,
- **dadurch gekennzeichnet, dass**
- der Gehalt an Stickstoff-abbauenden Bakterien untersucht wird.

7. Verfahren nach Anspruch 6,
- **dadurch gekennzeichnet, dass**
- der Gehalt an Nitrifikanten bestimmt wird.

8. Verfahren nach Anspruch 6 oder 7,
- **dadurch gekennzeichnet, dass**
- der Gehalt an Denitrifikanten bestimmt wird.

9. Verfahren nach einem der vorstehenden Ansprüche,
- **dadurch gekennzeichnet, dass**
- der Gehalt an fadenförmigen Bakterien und/oder an Bio-P-Anammox und/oder an Methanogenen bestimmt wird.

10. Verfahren nach einem der vorstehenden Ansprüche,
- **dadurch gekennzeichnet, dass**
- die Probe 10 bis 20 min, insbesondere etwa 15 min bestrahlt wird.

11. Verfahren nach einem der vorstehenden Ansprüche,
- **dadurch gekennzeichnet, dass**
- eine wässrige Probe untersucht wird.

12. Verfahren nach Anspruch 11,
- **dadurch gekennzeichnet, dass**
- es sich bei der wässrigen Probe um eine wässrige Lösung oder eine wässrige Suspension handelt.

13. Verfahren nach Anspruch 11 oder 12,
- **dadurch gekennzeichnet, dass**
- die wässrige Probe auf einen Trockensubstanzwert von 0,5 bis 5,0 g/l, insbesondere auf 2,0 g/l verdünnt wird.

14. Verfahren nach einem der vorstehenden Ansprüche,
- **dadurch gekennzeichnet, dass**
- die Probe einer Kläranlage, einer Biogasanlage oder einer sonstigen Industrieanlage entnommen wird.
